(19) Europäisches Patentamt
European Patent Office
Office européen des brevets

(11) Publication number: 0 041 871
B1

(12) # EUROPEAN PATENT SPECIFICATION

(45) Date of publication of patent specification: 21.03.84

(51) Int. Cl.³: C 07 C 69/54, C 07 C 67/28

(21) Application number: 81302579.8

(22) Date of filing: 10.06.81

(54) **Unsaturated ester polyether polyols and the use thereof as non-ionic surfactants.**

(30) Priority: 11.06.80 GB 8019125

(43) Date of publication of application: 16.12.81 Bulletin 81/50

(45) Publication of the grant of the patent: 21.03.84 Bulletin 84/12

(84) Designated contracting states: BE DE FR GB IT NL SE

(56) References cited:
DE-A- 2 643 701
US-A- 3 277 157

(73) Proprietor: BP Chemicals Limited
Britannic House Moor Lane
London, EC2Y 9BU (GB)

(72) Inventor: Biggin, Ian Stuart
BP Chemicals Limited Hayes Road Sully Penarth
South Glamorgan CF6 2YU Wales (GB)

(74) Representative: Harry, John et al
BP INTERNATIONAL LIMITED Patents and Licensing
Division Chertsey Road
Sunbury-on-Thames Middlesex TW16 7LN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

# Unsaturated ester polyether polyols and the use thereof as non-ionic surfactants

The present invention relates to novel unsaturated ester polyether polyols and to the use thereof as non-ionic surfactants.

Surfactants are commonly used in detergents, in emulsion polymerisation reactions and in the preparation of latices such as for instance in vinyl acrylic latices. Initially products derived from alkoxylated alkyl phenols, e. g. ethoxylated nonyl phenol, were used as surfactants. However, emulsions in which such surfactants were used lacked stability and expensive protective colloids had to be used to improve the stability of the emulsions. More recently, non-ionic surfactants based on the reaction products of olefin oxides and compounds containing reactive hydrogen atoms have been used. Typical examples of these non-ionic surfactants are the « Pluronics » (Regd. Trade Mark) and the « Pluriols » (Regd. Trade Mark) which are essentially block or random copolymers of polyoxyethylene and polyoxypropylene which have been reacted with compounds containing active hydrogen atoms. In these copolymers, the ethylene oxide part of the molecule is hydrophilic and the propylene oxide part of the molecule is hydrophobic. Such non-ionic surfactants are disclosed for example in British Patent Specification N$^{os}$ 731603 and 800159. In emulsion polymerisation these surfactants are soluble both in the monomer phase being polymerised and in the aqueous phase.

The surfactants therefore facilitate process variations and enable a wide range of products to be produced. However, the surfactant polyol molecules in the copolymer used as surfactant do not contain a suitable functional group which will enable them to be retained by the polymer, eg by co-polymerisation, and thus having to rely on a grafting mechanism for retention. The surfactant is therefore potentially liable to be extended by solvents inevitably present in or used in the preparation of such emulsions and may thus adversely affect the stability of the emulsions produced, especially during storage.

Compounds of increased functionality have been reported in Japanese Patent publication N° 7214079 (Toray Ind) in which methacrylic acid is reacted initially with polyethylene glycol and subsequently with polypropylene glycol. The resultant products are hydrophilic and are therefore watersoluble. This makes the product unsuitable for use as non-ionic surfactants, especially in emulsions. The water solubility can be reduced to a desired extent by using an inordinately long polypropylene glycol chain, eg having a molecular weight well above 1,000, as the final reactant. However, such a long chain polypropylene glycol creates problems during the alkoxylation stage because the chain tends to breakdown and gives rise to undesirable side reactions, especially in the presence of an alkoxylation catalyst.

It is an object of the present invention to produce a non-ionic surfactant in which the chain length of the hydrophobic polyalkylene glycol substantially reduced in order to achieve the desired reduction in water-solubility.

Accordingly, the present invention is a non-ionic surfactant of the general formula (I):

$$CH_2 = \underset{\displaystyle R_1}{\overset{|}{C}} - CO.(X)(\underset{\displaystyle R_2}{\overset{|}{C}H}.CH_2O)_m\ (CH_2.CH_2O)_nH \qquad (I)$$

wherein X is an organic moiety derived from an olefin oxide or a corresponding glycol having 3 to 8 carbon atoms, $R_1$ is H or a methyl group and $R_2$ is a methyl or an ethyl group, and each of m and n represent an integer from 2 to 20.

According to a further embodiment, the present invention relates to a process for producing non-ionic surfactants of the formula (I) comprising reacting a vinyl carboxylate of the formula (II)

$$CH_2 = C(R_1) — CO(X)H \qquad (II)$$

wherein X and $R_1$ have the same significance as in formula (I) above, initially with at least two moles of a hydrophobic olefin oxide containing 3 or 4 carbon atoms and subsequently with at least two moles of ethylene oxide at elevated temperature in the presence of an alkoxylation catalyst.

Thus the compounds from which the radical X of formula (I) and (II) is derived may be selected from low molecular weight compounds which do not contain more than 6 reactive hydrogen atoms. Specifically, these may be propylene glycol, 1,3-butylene glycol, 2,3-butylene glycol, 1,5-pentane diol, 1,6-hexane diol and phenyl-1,2-ethane diol.

In preparing the compounds of the present invention, the vinyl carboxylate may be either preformed or prepared by reacting a vinyl carboxylic acid with a compound containing at least two reactive hydrogen atoms. The vinyl carboxylic acid is preferably acrylic or methacrylic acid. The vinyl carboxylate is preferably a hydroxy alkyl acrylate or methacrylate, most preferably a hydroxypropyl acrylate or methacrylate. These may be prepared by known methods of esterification or condensation reactions between the vinyl carboxylic acid and either a corresponding glycol or olefin oxide. One such method is

disclosed in our granted European Patent Specification N° 0014041. It will be appreciated that if the vinyl carboxylic acid is reacted with an olefin oxide to form the ester, the olefin oxide does not actually contain reactive hydrogen atoms. However, the final vinyl carboxylate product has a structure in which the alcohol moiety of the ester is at least notionally derived from a glycol. The present invention embraces within its scope vinyl carboxylates derived in this manner. The vinyl carboxylate is then sequentially reacted with at least one mole of a hydrophobic olefin oxide and at least one mole of ethylene oxide in the presence of an alkoxylation catalyst. The precise amount of the hydrophobic olefin oxide and ethylene oxide used can be controlled to meet the characteristics such as hydrophilicity, hydrophobicity, molecular weight, physical properties and the like desired in the end product.

It is preferable to carry out the reaction in two stages. In the first stage, a block derivative of one of the hydrophobic olefin oxides is formed with the vinyl carboxylate. when this stage is completed the resultant product is reacted with ethylene oxide in a second stage. In forming a first stage block derivative, it is preferable to react the vinyl carboxylate with one of the hydrophobic olefin oxides by step-wise addition of the latter into the reaction mixture for ease of reaction. Whilst a preformed block of the polyoxyalkylene glycol may be used, this requires reaction conditions which are more severe than would be necessary for the stepwise build-up of the block derivative. When the desired amount of the hydrophobic olefin oxide has been reacted the first stage is complete. The product from the first stage is thereafter reacted in a second stage with ethylene oxide in a manner analogous to that of the first stage.

The oxyalkylation catalyst that is used for reacting the vinyl carboxylate with the olefin oxides is conventional. These catalysts may be acidic such as for instance boron trifluoride and tin chlorides, e. g. stannic chloride, or basic. Examples of basic catalysts include amines, alkalimetal hydroxides and alkali metal alcoholates. If amines are used as catalysts, they are unlikely to produce end products having a molecular weight above 600.

The concentration of catalyst used in any stage of the process will depend on the molecular weight of the product desired. If a relatively high molecular weight product is desired, eg around 2,500 preferably between 1,000 and 2,000, a relatively high catalyst concentration is used. Thus the catalyst concentration is suitably between 0.1 and 15 mole per cent, preferably between 1 and 15 mole per cent of boron trifluoride or stannic chloride based on the alkylene oxide.

The oxyalkylation is suitably carried out at a temperature between ambient and 170 °C, preferably between ambient and 125 °C depending upon the catalyst type used.

The reaction products are the mixed polyol ether esters of vinyl carboxylic acid. Specific examples of the products of the present invention are :

$$CH_2 = CH - CO(OC_3H_6O)(C_4H_8O)_m(C_2H_4O)_nH \qquad (III)$$

$$CH_2 = CH - CO(OC_3H_6O)(C_3H_6O)_m(C_2H_4O)_nH \qquad (IV)$$

$$CH_3$$
$$|$$
and
$$CH_2 = C - CO(OC_3H_6O)(C_3H_6O)_m(C_2H_4O)_nH \qquad (V)$$

$$CH_3$$
$$|$$
$$CH_2 = C - CO(OC_3H_6O)(C_4H_8O)_m(C_2H_4O)_nH \qquad (VI)$$

In formula (III)-(VI) the notations m and n represent integers from 2 to 20.

The products of the present invention may be used as non-ionic surfactants and as emulsifiers in emulsion polymerisation reactions eg in producing vinyl acrylic latices. The presence of a double bond in the vinyl grouping of these compounds enables them to react with other monomers and they are therefore not easily extracted by solvents. The vinyl double bond also enables them to be co-polymerised with other comonomers such as vinyl acetate, acrylates, methacrylates, acrylonitrile, acrylamide, styrene and vinylidene chloride. Other uses include those in the production of polyurethane foams, for removing soil from textile fabrics, and as plasticizers for a wide range of thermoplastic and thermosetting resins.

The present invention is further illustrated by the following Examples.

## Example 1

A mixture of hydroxypropyl methacrylate (2.5 moles) and boron trifluoride diethyl etherate (12.9 g) was added to a 2 litre glass reaction vessel fitted with condenser, stirrer and internal cooling coil. Propylene oxide (7.5 moles) was added gradually to the stirred mixture over 1.9 h, the temperature being maintained at 40°-50 °C throughout using cold water cooling. The mixture was stirred for a further 1 h to ensure essentially quantitative conversion of the propylene oxide prior to addition of 0.072 g of Curetard A (Registered Trade Mark). A mixture of ethylene oxide and nitrogen was then sparged into the reactor at

45°-60 °C, 16.8 moles of the oxide being taken up over 4.25 h. The alkoxylation catalyst was then deactivated with sodium carbonate (27 g) and the product filtered to remove solids.

The cloud point of a 1 % w/w aqueous solution of the product,

$$CH_2 = C(CH_3)CO.\ (O\overset{CH_3}{\overset{|}{C}}HCH_2O)(\overset{CH_3}{\overset{|}{C}}HCH_2O)_3(CH_2CH_2O)_{6.7}H,$$

was 15 °C.

## Example 2

Example 1 was repeated using the following reactant levels : hydroxypropyl methacrylate (2 moles), propylene oxide (12 moles), ethylene oxide (11.4 moles), boron trifluoride diethyletherate (11.5 g), Curetard A (Registered Trade Mark) (0.072 g) and sodium carbonate (24 g). The propylene oxide addition time, further first stage stirring time and ethylene oxide addition time were 2.3 h, 2.8 h and 3.5 h, respectively.

The cloud point of a 1 % w/w aqueous solution of the product,

$$CH_2 = C(CH_3)\ CO.(O\overset{CH_3}{\overset{|}{C}}HCH_2O)(\overset{CH_3}{\overset{|}{C}}HCH_2O)_6(CH_2CH_2O)_{5.7}H$$

was 8 °C.

## Comparative Test

In a comparative test not according to the invention, the procedure of Example 1 was repeated except that the starting material was hydroxyethylmethacrylate which was initially reacted with ethylene oxide and then with propylene oxide to obtain the following product.

$$CH_2 = C(CH_3)CO.(OCH_2CH_2O)(CH_2CH_2O)_6(\overset{CH_3}{\overset{|}{C}}H.CH_2O)_{3.7}H$$

This product had a cloud point of 31 °C thus showing it to be much more soluble than that in Example 1.

## Claims

1. A non-ionic surfactant of the general formula (I) :

$$CH_2 = \overset{R_1}{\overset{|}{C}} - CO.(X)(\overset{R_2}{\overset{|}{C}}H.CH_2O)_m\ (CH_2.CH_2O)_nH \qquad (I)$$

wherein X is an organic radical derived from an olefin oxide or a corresponding glycol having 3 to 8 carbon atoms, $R_1$ is an H atom or a methyl group and $R_2$ is a methyl or an ethyl group, and each of m and n represent an integer from 2 to 20.

2. A process for producing non-ionic surfactants of the formula (I) in Claim 1 comprising reacting a vinyl carboxylate of the formula (II) :

$$CH_2 = \overset{R_1}{\overset{|}{C}} - CO(X)H \qquad (II)$$

wherein X and $R_1$ have the same significance as in formula (I) above, initially with at least two moles of a hydrophobic olefin oxide containing 3 or 4 carbon atoms and subsequently with at least two moles of ethylene oxide at elevated temperature in the presence of an alkoxylation catalyst.

3. A non-ionic surfactant according to claim 1 wherein the radical X is derived from a compound selected from propylene glycol, 1,3-butylene glycol, 2,3-butylene glycol, 1,5-pentane diol, 1,6-hexane diol and phenyl-1,2-ethane diol.

4. A non-ionic surfactant according to any one of the preceding claims wherein the vinyl carboxylate is a hydroxy alkyl acrylate or a hydroxyalkyl methacrylate.

5. A non-ionic surfactant according to claim 4 wherein the vinyl carboxylate is a hydroxypropyl acrylate or a hydroxypropyl methacrylate.

6. A process for producing a vinyl carboxylate according to claim 2 wherein the non-ionic surfactant is prepared by forming a block derivative of the hydrophobic olefin oxide and the vinyl carboxylate in a first stage, and reacting the block derivative with ethylene oxide in the second stage.

7. A process according to claim 6 wherein the first stage block derivative is formed by reacting the vinyl carboxylate with one of the hydroprobic olefin oxides by step-wise addition of the latter into the reaction mixture.

8. A process according to any one of claims 2, 6 and 7 wherein the reaction between the vinyl carboxylate and the olefin oxides is carried out in the presence of an oxyalkylation catalyst selected from boron trifluoride, tin chlorides, amines, alkali metal hydroxides and alkali metal alcoholates.

9. A non-ionic surfactant selected from :

$$CH_2 = CH - CO(OC_3H_6O)(C_4H_8O)_m(C_2H_4O)_nH \qquad (III)$$

$$CH_2 = CH - CO(OC_3H_6O)(C_3H_6O)_m(C_2H_4O)_nH \qquad (IV)$$

$$CH_2 = \overset{CH_3}{\overset{|}{C}} - CO(OC_3H_6O)(C_3H_6O)_m(C_2H_4O)_nH \qquad (V)$$

$$CH_2 = \overset{CH_3}{\overset{|}{C}} - CO(OC_3H_6O)(C_4H_8O)_m(C_2H_4O)_nH \qquad (VI)$$

wherein the notations m and n in formulae (III)-(VI) represent integers from 2 to 20.

**Ansprüche**

1. Nichtionisches, oberflächenaktives Mittel der allgemeinen Formel (I) :

$$CH_2 = \overset{R_1}{\overset{|}{C}} - CO.(X)(\overset{R_2}{\overset{|}{CH}}.CH_2O)_m\ (CH_2.CH_2O)_nH \qquad (I)$$

in welcher X ein von einem Olefinoxyd oder einem entsprechenden Glycol mit 3 bis 8 Kohlenstoffatomen abgeleiteter organischer Rest, $R_1$ ein Wasserstoffatom oder eine Methylgruppe und $R_2$ eine Methyl- oder eine Äthylgruppe ist und m und n jeweils eine ganze Zahl von 2 bis 20 darstellen.

2. Verfahren zur Herstellung eines nichtionischen oberflächenaktiven Mittels der Formel I in Anspruch 1, wobei ein Vinylcarboxylat der Formel (II) :

$$CH_2 = \overset{R_1}{\overset{|}{C}} - CO(X)H \qquad (II)$$

worin X und $R_1$ die gleiche Bedeutung wie in Formel I besitzen, anfänglich mit zumindest zwei Molen eines hydrophoben Olefinoxyds mit 3 oder 4 Kohlenstoffatomen und danach mit mindestens 2 Molen Äthylenoxyd bei erhöhter Temperatur in Anwesenheit eines Alkoxylierungskatalysators umgesetzt wird.

3. Nichtionisches oberflächenaktives Mittel gemäß Anspruch 1, wobei der Rest X abgeleitet ist von einer Verbindung ausgewählt unter Propylenglycol, 1,3-Butylenglycol, 2,3-Butylenglycol, 1,5-Pentandiol, 1,6-Hexandiol und Phenyl-1,2-Ethandiol.

4. Nichtionisches oberflächenaktives Mittel gemäß einem der vorgehenden Ansprüche worin das Vinylcarboxylat ein Hydroxyalkylacrylat oder ein Hydroxyalkylmethacrylat ist.

5. Nichtionisches oberflächenaktives Mittel gemäß Anspruch 4, wobei das Vinylcarboxylat ein Hydroxypropylacrylat oder ein Hydroxypropylmethacrylat ist.

6. Verfahren zur Herstellung eines nichtionischen oberflächenaktiven Mittels gemäß Anspruch 2, wobei das nichtionische oberflächenaktive Mittel hergestellt wird durch Bildung eines Blockderivates des hydrophoben Olefinoxyds und des Vinylcarboxylats in einer ersten Stufe und Umsetzung des Blockderivats mit Äthylenoxyd in der zweiten Stufe.

7. Verfahren gemäß Anspruch 6, worin das Blockderivat der ersten Stufe gebildet wird durch Umsetzung des Vinylcarboxylats mit einem der hydrophoben Olefinoxyde durch stufenweise Zugabe des letzteren zu der Reaktionsmischung.

8. Verfahren gemäß einem der Ansprüche 2 und 7, wobei die Umsetzung zwischen dem Vinylcarboxylat und den Olefinoxyden durchgeführt wird in Anwesenheit eines oxyalkylierungskatalysators ausgewählt unter Bortrifluorid, Zinnchloriden, Aminen, Alkalimetallhydroxyden und Alkalimetallalkoholaten.

9. Nichtionisches oberflächenaktives Mittel ausgewählt unter

$$CH_2 = CH - CO(OC_3H_6O)(C_4H_8O)_m(C_2H_4O)_nH \qquad (III)$$

$$CH_2 = CH - CO(OC_3H_6O)(C_3H_6O)_m(C_2H_4O)_nH \qquad (IV)$$

$$CH_2 = \overset{\displaystyle CH_3}{\overset{|}{C}} - CO(OC_3H_6O)(C_3H_6O)_m(C_2H_4O)_nH \qquad (V)$$

$$CH_2 = \overset{\displaystyle CH_3}{\overset{|}{C}} - CO(OC_3H_6O)(C_4H_8O)_m(C_2H_4O)_nH \qquad (VI)$$

wobei die Bezeichnungen m und n in den Formeln (III) bis (VI) ganze Zahlen von 2 bis 20 darstellen.

**Revendications**

1. Surfactif non ionique de formule générale (I) :

$$CH_2 = \overset{\displaystyle R_1}{\overset{|}{C}} - CO.(X)(\overset{\displaystyle R_2}{\overset{|}{CH}}.CH_2O)_m\ (CH_2.CH_2O)_nH \qquad (I)$$

dans laquelle X est un radical organique provenant d'un oxyde d'oléfine ou d'un glycol correspondant ayant 3 à 8 atomes de carbone, $R_1$ représente un atome d'hydrogène ou un groupe méthyle et $R_2$ représente un groupe méthyle ou éthyle, et chacun des indices m et n représente un nombre entier valant 2 à 20.

2. Procédé pour produire des surfactifs non ioniques répondant à la formule (I) de la revendication 1, comprenant la réaction d'un vinyl carboxylate de formule (II) :

$$CH_2 = \overset{\displaystyle R_1}{\overset{|}{C}} - CO(X)H \qquad (II)$$

(dans laquelle X et $R_1$ ont le même sens que pour la formule (I) ci-dessus), initialement avec au moins 2 moles d'un oxyde d'oléfine hydrophobe contenant 3 ou 4 atomes de carbone puis avec au moins 2 moles d'oxyde d'éthylène à température élevée en présence d'un catalyseur d'alcoxylation.

3. Surfactif non ionique selon la revendication 1, dans lequel le radical X provient d'un composé choisi parmi le propylène glycol, le butylène glycol-1,3, le butylène glycol-2,3, le pentane diol-1,5, l'hexane diol-1,6 et le phényl éthane diol-1,2.

4. Surfactif non ionique selon l'une quelconque des revendications précédentes, dans lequel le vinyl carboxylate est un acrylate d'hydroxy alkyle ou un méthacrylate d'hydroxy alkyle.

5. Surfactif non ionique selon la revendication 4, dans lequel le vinyl carboxylate est un acrylate d'hydroxypropyle ou un méthacrylate d'hydroxypropyle.

6. Procédé pour produire un surfactif non ionique selon la revendication 2, dans lequel on prépare le surfactif non ionique en formant dans une première étape un dérivé séquencé de l'oxyde d'oléfine hydrophobe et du vinyl carboxylate, et en faisant réagir dans la seconde étape le dérivé séquencé avec de l'oxyde d'éthylène.

7. Procédé selon la revendication 6, dans lequel on forme le dérivé séquencé de la première étape en faisant réagir le vinyl carboxylate avec l'un des oxydes d'oléfine hydrophobe par l'addition progressive de ce dernier au mélange réactionnel.

8. Procédé selon l'une quelconque des revendications 2, 6 et 7, dans lequel on effectue la réaction entre le vinyl carboxylate et les oxydes d'oléfines en présence d'un catalyseur d'oxyalkylation choisi parmi du trifluorure de bore, des chlorures de l'étain, des amines, des hydroxydes de métaux alcalins et des alcoolates de métaux alcalins.

9. Surfactif non ionique choisi parmi :

$$CH_2 = CH - CO(OC_3H_6O)(C_4H_8O)_m(C_2H_4O)_nH \qquad (III)$$

$$CH_2 = CH - CO(OC_3H_6O)(C_3H_6O)_m(C_2H_4O)_nH \qquad (IV)$$

$$CH_2 = \overset{\displaystyle CH_3 \atop |}{C} - CO(OC_3H_6O)(C_3H_6O)_m(C_2H_4O)_nH \qquad (V)$$

$$CH_2 = \overset{\displaystyle CH_3 \atop |}{C} - CO(OC_3H_6O)(C_4H_8O)_m(C_2H_4O)_nH \qquad (VI)$$

dans lequel les indices m et n des formules (III) à (VI) représentent des nombres entiers valant 2 à 20.